(19) Europäisches Patentamt
European Patent Office
Office européen des brevets

(11) EP 3 389 610 B1

(12) **EUROPEAN PATENT SPECIFICATION**

(45) Date of publication and mention of the grant of the patent:
**17.04.2024 Bulletin 2024/16**

(21) Application number: **16820508.6**

(22) Date of filing: **15.12.2016**

(51) International Patent Classification (IPC):
***A61K 8/34*** *(2006.01)* ***A61K 8/44*** *(2006.01)*
***A61K 8/46*** *(2006.01)* ***A61Q 5/02*** *(2006.01)*
***A61Q 5/12*** *(2006.01)*

(52) Cooperative Patent Classification (CPC):
**A61K 8/463; A61K 8/345; A61K 8/442; A61Q 5/02; A61Q 5/12**

(86) International application number:
**PCT/US2016/066752**

(87) International publication number:
**WO 2017/106398 (22.06.2017 Gazette 2017/25)**

(54) **HAIR CLEANSING COMPOSITION**

HAARWASCHMITTEL

COMPOSITION DE NETTOYAGE DE CHEVEUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.12.2015 US 201562267492 P**
**21.10.2016 US 201662411051 P**

(43) Date of publication of application:
**24.10.2018 Bulletin 2018/43**

(73) Proprietor: **The Procter & Gamble Company**
**Cincinnati, OH 45202 (US)**

(72) Inventors:
- **ZHAO, Jean, Jianqun**
  **Cincinnati**
  **Ohio 45202 (US)**
- **CONSTANTINIDES, Ioannis, Constantine**
  **Cincinnati**
  **Ohio 45202 (US)**
- **HUTCHINS, Thomas, Allen**
  **Cincinnati**
  **Ohio 45202 (US)**
- **HURLEY, Brian, Michael**
  **Cincinnati**
  **Ohio 45202 (US)**
- **GLENN JR., Robert, Wayne**
  **Cincinnati**
  **Ohio 45202 (US)**

(74) Representative: **P&G Patent Germany**
**Procter & Gamble Service GmbH**
**Sulzbacher Straße 40**
**65824 Schwalbach am Taunus (DE)**

(56) References cited:
**EP-A1- 2 042 216** **WO-A1-01/42409**
**WO-A1-2006/113117** **WO-A1-2016/172405**
**WO-A2-02/092050** **DE-A1- 4 315 396**
**JP-A- 2012 001 597** **US-A- 5 747 436**
**US-A1- 2001 056 049** **US-A1- 2006 183 662**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 389 610 B1

## Description

FIELD OF THE INVENTION

The present invention relates to a deep cleansing hair care composition and method that provide good in-use experience and superior hair benefits when dry.

BACKGROUND OF THE INVENTION

Typically, clarifying shampoos are employed to provide a high level of cleaning for hair, good lather and clean rinse feel. However, such shampoos are perceived to strip the natural moisturizers from the hair fibers, as they result in high wet hair friction that is translated into poor wet feel and high dry hair friction that is translated into poor, non-moisturized dry hair feel. The poor wet feel and high dry hair friction are particularly noticeable in the case of consumers that have chemically or physically damaged hair resulting from permanent or semi-permanent styling treatments, oxidative coloring treatments, thermal treatments, etc. To remedy the poor wet feel and the high dry hair friction, consumers often turn to traditional conditioning shampoos which can provide good wet and dry hair feel. However, many of these products do not possess strong cleansing ability, they generate lower volume of lather during use, they do not provide clean rinse feel and they can allow more soils, sebum and other residues to remain on the hair after shampooing.

EP 2 042 216 A1 is related to body and hair cleansing products especially intended for infants and kids, only containing constituents used in foodstuff, especially additives appearing on the E list.

JP 2012 001597 A refers to a detergent composition having extremely excellent detergency and having a bicontinuous smear emulsion phase.

US 2001/056049 A1 sets out an aqueous detergent composition, which is in the form of a thickened, mobile fluid, comprising foaming detergent and a polymer or polymer mixture which is capable of forming a reversible gel, which polymer or mixture is present in the composition as a multiplicity of individual gel particles.

WO 02/092050 A2 concerns a disposable substantially damp cleansing article is disclosed having a cleansing composition impregnated onto a flexible substrate such as a non-woven cloth.

WO 01/42409 A1 is high content glycerol liquid compositions comprising N-acyl amino acids and/or salts and defined sulfosuccinic acid monoesters.

US 2006/183662 A1 refers to a disposable substantially damp cleansing article is disclosed having a cleansing composition impregnated onto a flexible substrate such as a non-woven cloth.

DE 43 15 396 A1 is related to cosmetic hair treatment compositions having a wet combability of the hair enhancing additive.

US 5,747,436 A is providing an effective conditioning shampoo composition which is free of conditioning amounts of silicone conditioning agents.

WO 2016/172404 A1 refers to a foamable hair care composition comprising an anionic surfactant, a co-surfactant. a viscosity reducing agent, and a cationic polymer having a weight average molecular weight of less than 1,000,000 g/mol.

In addition, the conditioning agents themselves can contribute to the feeling of residue that is added on the hair surface during the shampoo and/or conditioner process that they may accumulate in each cycle. This can result in hair weigh-down with low volume that is perceived as greasy, and that may also be perceived by the consumer as lanky, non-bouncy and difficult to style. Thus, there is a need for the development of shampoo compositions that provide strong cleansing ability and at the same time contribute to high hair volume, good wet and dry hair feel, and bouncy, easy to style hair.

Described herein is a deep cleansing hair care composition that enables a thorough removal of soils such as sebum, conditioning, styling and other residues from hair surface in order to achieve clean hair with high hair volume, smooth dry hair feel and elastic hair fibers that impart bouncy hair. In addition, the hair care composition described herein results in an excellent in-use experience by providing rich lather, good wet conditioning feel, and a clean rinse feel. Additionally, the hair care composition is stable and uniform and can be clear, translucent or opaque.

SUMMARY OF THE INVENTION

The invention relates to a hair care composition comprising: from 5 to 40 weight % of the hair care composition one or more detersive surfactants; wherein, the detersive surfactant comprises less than 10 weight % of the hair care composition of a linear anionic surfactant selected from the group consisting of:

(1) alkyl sulfates

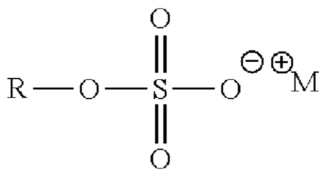

Alkyl sulfates

where R is a linear $C_8$-$C_{24}$ alkyl and $M^+$ is monovalent cation;
(2) alkyl ether sulfates

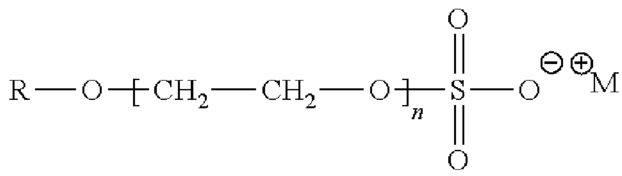

Alkyl ether sulfates

where R is a linear $C_8$-$C_{24}$ alkyl, n=1-2, and $M^+$ is monovalent cation;
(3) and mixture thereof,

wherein one of the detersive surfactant is a branched anionic surfactant selected from the group consisting of:

(1) alkyl sulfates

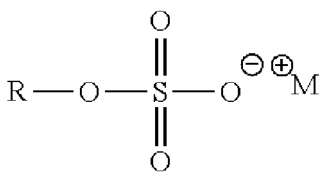

Alkyl sulfates

where R is a branched $C_8$-$C_{24}$ alkyl and $M^+$ is monovalent cation;
(2) alkyl ether sulfates

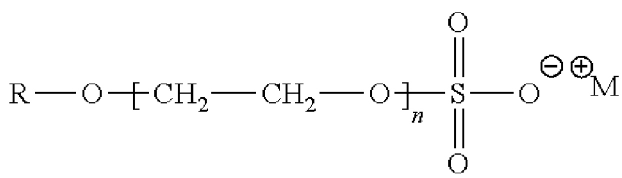

Alkyl ether sulfates

where R is a branched $C_8$-$C_{24}$ alkyl, n=1-2, and $M^+$ is monovalent cation; and mixtures thereof;

and a carrier comprising one or more polyols and water, wherein the carrier comprises from 20 to 80 weight % of the hair care composition of one or more polyols, and from 9% to 75% by weight of the hair care composition of water; and wherein the weight ratio of one or more polyols to water is 0.4 or higher.

DETAILED DESCRIPTION OF THE INVENTION

While the specification concludes with claims which particularly point out and distinctly claim the invention, it

is believed that the provided invention will be better understood from the following description.

All percentages and ratios used herein are by weight of the total composition, unless otherwise designated. All measurements are understood to be made at ambient conditions, where "ambient conditions" means conditions at 25 °C, under one atmosphere of pressure, and at 50% relative humidity (RH), unless otherwise designated. All numeric ranges are inclusive of narrower ranges; delineated upper and lower range limits are combinable to create further ranges not explicitly delineated. All numerical amounts are understood to be modified by the word "about" unless otherwise specifically indicated. All weights and % weights as they pertain to listed ingredients are based on the active level and do not include carriers or by-products that may be included in commercially available materials, unless otherwise specified. The number of significant digits conveys neither a limitation on the indicated amounts nor on the accuracy of the measurements. The term "molecular weight" or "M.Wt." as used herein refers to the weight average molecular weight unless otherwise stated. The weight average molecular weight may be measured by gel permeation chromatography. "QS" means sufficient quantity for 100%.

DEFINITIONS

"Dermatologically acceptable" means that the compositions or components described are suitable for use in contact with human skin tissue without undue toxicity, incompatibility, instability, allergic response, and the like.

"Safe and effective amount" means an amount of a compound or composition sufficient to significantly induce a positive benefit.

"Soluble" means at least 0.1 g of solute dissolves in 100 ml of solvent, at 25 °C and 1 atm of pressure.

The term "substantially free from" or "substantially free of" as used herein means less than 1%, or less than 0.8%, or less than 0.5%, or less than 0.3%, or 0%, or from 1% to 0%, or 0.8% to 0%, or 0.3% to 0% by total weight of the composition.

"Hair," as used herein, means mammalian hair including scalp hair, facial hair and body hair, particularly hair on the human head and scalp.

"Cosmetically acceptable," as used herein, means that the compositions, formulations or components described are suitable for use in contact with human keratinous tissue without undue toxicity, incompatibility, instability, allergic response, and the like. All compositions described herein which have the purpose of being directly applied to keratinous tissue are limited to those being cosmetically acceptable.

"Derivatives," as used herein, includes but is not limited to, amide, ether, ester, amino, carboxyl, acetyl, acid, salt and/or alcohol derivatives of a given compound.

"Polymer," as used herein, means a chemical formed from the polymerisation of two or more monomers. The term "polymer" as used herein shall include all materials made by the polymerisation of monomers as well as natural polymers. Polymers made from only one type of monomer are called homopolymers. Polymers made from two or more different types of monomers are called copolymers. The distribution of the different monomers can be calculated statistically or block-wise - both possibilities are suitable for the present invention. Except if stated otherwise, the term "polymer" used herein includes any type of polymer including homopolymers and copolymers.

SHAMPOO COMPOSITION

Typical clarifying shampoos, which contain minimal conditioning agents, provide superior cleansing via removal of soils, sebum and other residues. However, they are perceived as hair stripping, with an in-use wet feel that does not provide the signal of conditioning. In addition, the hair after washing with clarifying shampoo is not smooth. The hair care compositions described herein which contain a carrier, a significant portion (20% to 80%) of which is a water-miscible polyol, such as glycerin, are able to minimize undesirable hair stripping. These compositions not only provide superior removal of soils, but also show excellent lather quality and clean rinse feel. The polyols may contribute to superior cleansing by reducing the size of the surfactant micelle structure. This, in turn, can result in rapid transfer of the micelles on hair fiber surfaces, and rapid removal of soils from these surfaces. It is surprisingly observed that the lather created during the cleansing process of the inventive compositions is creamy and resilient, providing slippery, conditioning feel. The superior cleansing is associated with volume expansion of the dry hair. It is also surprising that the inventive compositions show larger hair expansion than traditional clarifying shampoos (see results in Table 1 for Ex 1 to Ex 3 versus Comparative Ex 1 and Comparative Ex 2). Additionally, the friction of dry hair that has been washed by the inventive compositions is below the expected level (resulting in hair smooth feel), as the corresponding surfaces are substantially free from oils, sebum and other hydrophobic materials. This may be the result of the plasticizing effect of the polyol (such as glycerin) on keratin protein and/or the expected higher water absorption of moisture in hair fibers because of the presence of residual amount of glycerin.

Described herein is a shampoo composition comprising a detersive surfactant, and an aqueous carrier comprising water and polyol, wherein the weight ratio of polyol to water is from 0.4 or higher, wherein the polyol content is

from 20 to 80 weight % of the composition, and wherein the water content is from 9% to 75% by weight of the composition.

The hair care composition may comprise from 4 to 40 weight % of one or more detersive surfactant. The hair care composition may comprise from 0 to 10 weight percent of alkyl sulfates with linear alkyl group. An alkyl sulfate having a linear alkyl group is a sulfate with an alkyl group with a formula of CH3(CH2)n-, wherein each carbon is bound to two neighbors and two hydrogen atoms, except the terminal carbon which is bound to three hydrogen atoms

The hair care composition may further comprise a cationic polymer.

The shampoo composition delivers consumer desired cleansing power (low residues on hair surface) in addition to good lather, good conditioning wet feel, clean rinse feel, high hair volume, smooth non-coated dry hair feel, and moisturized bouncy hair that is easy to style.

Water is a small molecule that moves in and out of damaged hair cuticles depending on the environment change. It can weaken hair fibers when wet, it can make hair fibers brittle when dry and it can cause hair color to fade by carrying color pigment out of hair. Polyols such as glycerin adds elasticity to damaged hair fibers and results in hair that is less sensitive to environment changes.

Shampoos with high levels of polyol such as glycerin can also affect surfactant packing. The micelles in the hair care composition are more segregated and therefore transport onto the hair surface quicker which can result in improved soil removal.

Typically, the viscosity of an aqueous solution is increased when the glycerin content is increased (Segur, J.B. and Oberstar, H., Ind. & Eng. Chem., 43: 2117-2120, 1951). However, the viscosity of the hair care composition of the present invention decrease when glycerin content is increased in the glycerin and water mixture carrier. Consumers traditionally prefer products with a viscosity of 1000 cps to 15,000 cps which result in enough viscosity so that they do not immediately run out of the hand after dispensing; however, too viscous products are difficult to spread onto the hair.

Consumer testing indicates that the hair care compositions described herein deliver soft and moisturized feel on damaged hair. Additionally, consumer testing indicates the hair care compositions described herein deliver one or more consumer benefits including, but not limited to rich and creamy lather, deep clean, scalp comfort, volume and shine.

## A. DETERSIVE SURFACTANT

The shampoo composition comprises one or more detersive surfactants, which provides cleaning performance to the composition. The one or more detersive surfactants in turn may comprise anionic surfactants, amphoteric or zwitterionic surfactants, nonionic surfactants or mixtures thereof. Various examples and descriptions of detersive surfactants are set forth in U.S. Patent No. 6,649,155; U.S. Patent Application Publication No. 2008/0317698; and U.S. Patent Application Publication No. 2008/0206355.

The concentration of the detersive surfactant component in the shampoo composition should be sufficient to provide the desired cleaning and lather performance. Generally, ranges are from 5 wt% to 40 wt%, from 5 wt% to 35 wt%, from 8 wt% to 35 wt%, from 10 wt% to 30 wt%, from 15 wt% to 25%, from 10 wt% to 20 wt% by weight of the composition.

The total surfactants can include, but are not limited to nonionic, amphoteric, branched anionic surfactants, linear anionic surfactants and combinations thereof.

### 1. Branched Anionic Surfactant

Suitable branched anionic surfactant, with a tail having a branched alkyl chain with 8 carbon atoms or higher, include, but are not limited to the following branched surfactants: sodium undecyl sulfate, sodium trideceth sulfate, sodium tridecyl sulfate, sodium C8- 13 alkyl sulfate, sodium C8-15 alkyl sulfate, sodium C8-18 alkyl sulfate, sodium C8-13 pareth sulfate, sodium C8-13 pareth-n sulfate, sodium C8-14 pareth-n sulfate, and combinations thereof. Other salts of all the aforementioned surfactants are useful, such as TEA, DEA, ammonia, potassium salts. Useful alkoxylates include the ethylene oxide, propylene oxide and EO/PO mixed alkoxylates. Phosphates, carboxylates and sulfonates prepared from branched alcohols are also useful anionic branched surfactants. Branched surfactants can be derived from synthetic alcohols such as the primary alcohols from the liquid hydrocarbons produced by Fischer-Tropsch condensed syngas, for example SafolTM 23 Alcohol available from Sasol North America, Houston, Tex.; from synthetic alcohols such as NeodolTM 23 Alcohol available from Shell Chemicals, USA; from synthetically made alcohols such as those described in U.S. Pat. No. 6,335,312 issued to Coffindaffer, et al on Jan. 1, 2002. Suitable examples of alcohols are SafolTM 23 and NeodolTM 23. Suitable examples of alkoxylated alcohols are SafolTM 23-3 and NeodolTM 23-3. Sulfates can be prepared by conventional processes to high purity from a sulfur based SO3air stream process, chlorosulfonic acid process, sulfuric acid process, or Oleum process. Preparation via air stream in a falling film reactor is a preferred sulfation process. The anionic surfactant may also be STnS, wherein n can define average moles of ethoxylation. n can range from 0 to 3.5, from 0.5 to 3.5, from 1.1 to 3.

2. Linear Anionic Surfactant

Suitable linear anionic detersive surfactant components for use in the composition herein include those which are known for use in hair care or other personal care shampoo compositions. The anionic detersive surfactant may be a combination of sodium lauryl sulfate and sodium laureth-n sulfate. Alternatively, the anionic detersive surfactant can be sodium laureth sulfate with an average of one mole ethoxylate. The concentration of the anionic surfactant component in the composition should be sufficient to provide the desired cleaning and lather performance.

Anionic surfactants suitable for use herein include alkyl sulfates and alkyl ether sulfates of the formula $ROSO_3M$ and $RO(C_2H_4O)_xSO_3M$, wherein R is alkyl or alkenyl of from 8 to 18 carbon atoms, x is 1 to 10, and M is a water-soluble cation such as ammonium, sodium, potassium, and triethanolamine cation or salts of the divalent magnesium ion with two anionic surfactant anions . The alkyl ether sulfates may be made as condensation products of ethylene oxide and monohydric alcohols having from 8 to 24 carbon atoms. The alcohols can be derived from fats such as coconut oil, palm oil, palm kernel oil, or tallow, or can be synthetic.

Table 1: Examples of Typical Alkyl Sulfates and Alky Ether Sulfates

| Surfactant | Supplier | Activity | SLS | SLE1S | SLE2S | SLE3S | SLE>3S |
|---|---|---|---|---|---|---|---|
| Sodium Lauryl Sulfate | Stepan STEaL SLS | 29% by weight | 100 | 0 | 0 | 0 | 0 |
| Sodium Laureth-1 Sulfate | Stepan STEOL SLES-1 | 26% by weight | 45.5 | 26.3 | 11.8 | 0.07 | 16.33 |
| Sodium Laureth-3 Sulfate | Stepan STEOL SLES-3 | 28% by weight | 18 | 16.7 | 12.6 | 12.4 | 40.30 |

Some non-limiting examples of linear anionic surfactants are:

Alkyl Sulfates

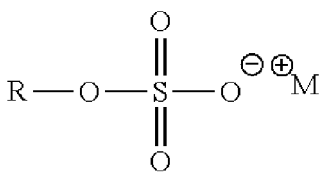

Alkyl sulfates

where R is $C_8$-$C_{18}$ alkyl (linear, saturated or unsaturated) or mixtures thereof and $M^+$ is monovalent cation. Examples include sodium lauryl sulfate (where R is $C_{12}$ alkyl and $M^+$ is $Na^+$), ammonium lauryl sulfate (where R is $C_{12}$ alkyl and $M^+$ is $NH_3{}^+$), and sodium coco-sulfate (where R is coconut alkyl and $M^+$ is $Na^+$);

Alkyl Ether Sulfates

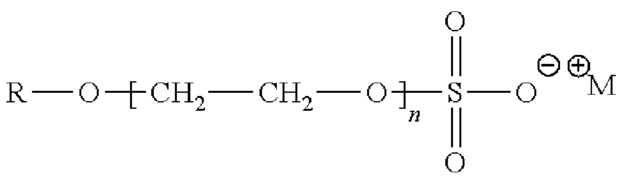

Alkyl ether sulfates

where R is $C_8$-$C_{18}$ alkyl (linear, saturated or unsaturated) or mixtures thereof, n=1-12, and $M^+$ is monovalent cation. Examples include sodium laureth sulfate (where R is $C_{12}$ alkyl and $M^+$ is $Na^+$, n=1-3), ammonium laureth sulfate (where R is $C_{12}$ alkyl, $M^+$ is $NH_3{}^+$, n=1-3), and Sodium trideceth sulfate (where R is $C_{13}$ alkyl, $M^+$ is $Na^+$, and n=1-4);

Some non-limiting examples of sulfonate surfactants are:

Alkyl glyceryl ether sulfonates:

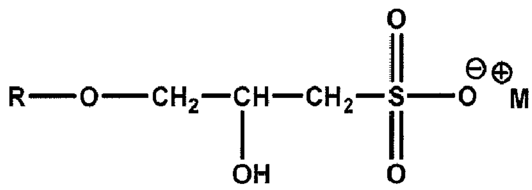

where R = $C_8$ - $C_{18}$ alkyl (linear, saturated or unsaturated) or mixtures thereof and $M^+$ = monovalent cation, such as Sodium Cocoglyceryl Ether Sulfonate
(R = coco alkyl, $M^+$ = $Na^+$);

Alpha olefin sulfonates prepared by sulfonation of long chain alpha olefins. Alpha olefin sulfonates consist of mixtures of alkene sulfonates,

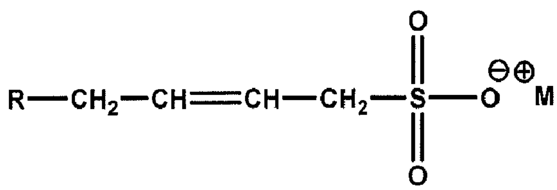

where R = $C_4$ - $C_{18}$ alkyl or mixtures thereof and $M^+$ = monovalent cation;

Hydroxyalkyl sulfonates,

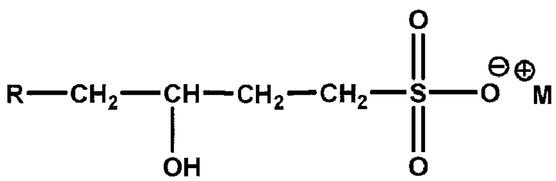

where R = $C_4$ - $C_{18}$ alkyl or mixtures thereof and $M^+$ = monovalent cation. Examples include Sodium C12-14 Olefin Sulfonate (R = $C_8$ - $C_{10}$ alkyl, $M^+$ = $Na^+$) and Sodium C 14-16 Olefin Sulfonate (R = $C_{10}$ - $C_{12}$ alkyl, $M^+$ = $Na^+$).

Examples of additional anionic surfactants suitable for use herein include, but are not limited to, ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium laureth sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, monoethanolamine cocoyl sulfate, sodium trideceth-1 sulfate, sulfate, sodium trideceth-2 sulfate, sulfate, sodium trideceth-3 sulfate, sodium tridecyl sulfate, sodium methyl lauroyl taurate, sodium methyl cocoyl taurate, sodium lauroyl isethionate, sodium cocoyl isethionate, sodium laurethsulfosuccinate, sodium laurylsulfosuccinate, sodium tridecyl benzene sulfonate, sodium dodecyl benzene sulfonate, and mixtures thereof.

Still other suitable anionic surfactants are the reaction products of fatty acids esterified with isethionic acid and neutralized with sodium hydroxide. Other similar anionic surfactants are described in U.S. Patent Nos. 2,486,921; 2,486,922; and 2,396,278. If the hair care composition comprises an anionic linear alkyl sulfate/linear alkyl ether sulfate at a level exceeding 10%, the hair care composition may further comprise from 1% to 15%, from 2% to 15%, from 2% to 10%, from 2% to 9%, from 2% to 8.5%, from 2% to 8% by weight of the hair care composition of an additional surfactant. The additional surfactant can be chosen from the group including amphoteric surfactant, zwitterionic surfactant, nonionic surfactant, and mixtures thereof. The ratio of anionic surfactant to additional surfactant is from 0.3 to 6; from 0.3 to 5.5, from 0.3 to 5, from 0.5 to 5.

Amphoteric detersive surfactants suitable for use in the shampoo composition include those surfactants broadly

described as derivatives of aliphatic secondary and tertiary amines in which the aliphatic radical can be straight or branched chain and wherein one of the aliphatic substituents contains from 8 to 18 carbon atoms and one contains an anionic group such as carboxy, sulfonate, sulfate, phosphate, or phosphonate. Exemplary amphoteric detersive surfactants for use in the present shampoo composition include cocoamphoacetate, cocoamphodiacetate, lauroamphoacetate, lauroamphodiacetate, and mixtures thereof.

Zwitterionic detersive surfactants suitable for use in the shampoo composition include those surfactants broadly described as derivatives of aliphatic quaternary ammonium, phosphonium, and sulfonium compounds, in which the aliphatic radicals can be straight or branched chain, and wherein one of the aliphatic substituents contains from 8 to 18 carbon atoms and one contains an anionic group such as carboxy, sulfonate, sulfate, phosphate or phosphonate. Zwitterionics such as betaines may be selected.

Non limiting examples of other anionic, zwitterionic, amphoteric or optional additional surfactants suitable for use in the shampoo composition are described in McCutcheon's, Emulsifiers and Detergents, 1989 Annual, published by M. C. Publishing Co., and U.S. Patent Nos. 3,929,678, 2,658,072; 2,438,091; 2,528,378. Non limiting examples of suitable zwitterionic or amphoteric surfactants are described in U.S. Patent Nos. 5,104,646 and 5,106,609.

Non-ionic detersive surfactants suitable for use in the shampoo composition are selected from the group consisting of: Cocamide, Cocamide Methyl MEA, Cocamide DEA, Cocamide MEA, Cocamide MIPA, Lauramide DEA, Lauramide MEA, Lauramide MIPA, Myristamide DEA, Myristamide MEA, PEG-20 Cocamide MEA, PEG-2 Cocamide, PEG-3 Cocamide, PEG-4 Cocamide, PEG-5 Cocamide, PEG-6 Cocamide, PEG-7 Cocamide, PEG-3 Lauramide, PEG-5 Lauramide, PEG-3 Oleamide, PPG-2 Cocamide, PPG-2 Hydroxyethyl Cocamide, and mixtures thereof.

## B. AQUEOUS CARRIER

The shampoo composition comprises an aqueous carrier which is a mixture of water and a polyol. Suitable polyols include glycerin or ethylene glycol, propylene glycol, di-propylene glycol and mixtures thereof. The polyol may be glycerin. The water content of the composition is from 9 % to 75%, from 10% to 65%, from 10% to 55%, from 10% to 45%, from 10% to 40%, and from 10% to 35% by weight of the composition. The polyol content is 20% to 80%, from 25% to 75%, or from 30% to 70%, from 35% to 70%, from 40% to 70% by weight of the total composition. The weight ratio of polyol to water is from 0.4 to 7, from 0.4 to 6.5, from 0.4 to 6, from 0.4 to 5.5, and from 0.5 to 5.5.

Suitable polyols also include water-miscible organic solvent such as propylene glycol, di-propylene glycol, and ethylene glycol.

The aqueous carrier may also include lower alcohols. The lower alcohols useful herein are monohydric alcohols having 1 to 6 carbons, in one aspect, ethanol and isopropanol.

In one aspect, the carrier may comprise other water-miscible or immiscible solvents with minimal or no significant concentrations of organic solvent, except as otherwise incidentally incorporated into the composition as minor ingredients of other components.

## C. CATIONIC POLYMER

The shampoo composition comprises one or more a cationic polymers. The hair care composition can comprises from 0.05 weight % to 2 weight % of one or more cationic polymers, from 0.05 weight % to 1.5 weight % of one or more cationic polymers, alternatively from 0.05 weight % to 1 weight % of one or more cationic polymers.

The polymer can include at least one of (a) a cationic guar polymer, (b) a cationic non-guar galactomannan polymer, (c) a cationic starch polymer, (d) a cationic copolymer of acrylamide monomers and cationic monomers, (e) a synthetic cationic polymer, (f) a cationic cellulose polymer or (g) a mixture of such polymers. The molecular weight of the cationic polymer can be from 1,000 to 10,000,000 and its charge density can be between 0.1 meq/g to 7 meq/g. The molecular weight of the cationic polymer can be from 500,000 to 900,000, from 500,000 to 800,000, and/or from 250,000 to 800,000.

### (a) Cationic guar polymer

The dispersion composition may comprise a cationic guar polymer, which is a cationically substituted galactomannan (guar) gum derivatives. Guar gum for use in preparing these guar gum derivatives is typically obtained as a naturally occurring material from the seeds of the guar plant. The guar molecule itself is a straight chain mannan, which is branched at regular intervals with single membered galactose units on alternative mannose units. The mannose units are linked to each other by means of $\beta$(1-4) glycosidic linkages. The galactose branching arises by way of an $\alpha$(1-6) linkage. Cationic derivatives of the guar gums are obtained by reaction between the hydroxyl groups of the polygalactomannan and reactive quaternary ammonium compounds.

The cationic guar polymer may be formed from quaternary ammonium compounds. Suitable cationic guar

polymers include cationic guar gum derivatives, such as guar hydroxypropyltrimonium chloride. The cationic guar polymer may be a guar hydroxypropyltrimonium chloride. Specific examples of guar hydroxypropyltrimonium chlorides include the Jaguar® series commercially available from Rhone-Poulenc Incorporated, for example Jaguar® C-500, commercially available from Rhodia. Jaguar® C-500 has a charge density of 0.8 meq/g and a M.Wt. of 500,000 g/mole. Jaguar® C-17, which has a cationic charge density of 0.6 meq/g and a M.Wt. of 2.2 million g/mol and is available from Rhodia Company. Jaguar® C 13S which has a M.Wt. of 2.2 million g/mol and a cationic charge density of 0.8 meq/g (available from Rhodia Company). Other suitable guar hydroxypropyltrimonium chloride are: guar hydroxypropyltrimonium chloride which has a charge density of 1.1 meq/g and a M.Wt. of 500,000 g/mole is available from ASI, a charge density of 1.5 meq/g and a M.Wt. of 500,000 g/mole is available from ASI. Other suitable guar hydroxypropyltrimonium chloride are: Hi-Care 1000, which has a charge density of 0.7 meq/g and a M.Wt. of 600,000 g/mole and is available from Rhodia; N-Hance 3269 and N-Hance 3270, which has a charge density of 0.7 meq/g and a M.Wt. of 425,000 g/mole and is available from ASI; N-Hance 3196, which has a charge density of 0.8 and a M. Wt. Of 1,100,000 g/ mole and is available from ASI. AquaCat CG518 has a charge density of 0.9 meq/g and a M.Wt. of 50,000 g/mole and is available from ASI. BF-13, which is a borate (boron) free guar of charge density of 1.1 meq/g and M. W.t of 800,000 and BF-17, which is a borate (boron) free guar of charge density of 1.7 meq/g and M. W.t of 800,000 both available from ASI.

(b) Cationic Non-Guar Galactomannan Polymers

The dispersion compositions of the present invention comprise a galactomannan polymer derivative having a mannose to galactose ratio of between 5:1 and 1:1 on a monomer to monomer basis, the galactomannan polymer derivative selected from the group consisting of a cationic galactomannan polymer derivative and an amphoteric galactomannan polymer derivative having a net positive charge. As used herein, the term "cationic galactomannan" refers to a galactomannan polymer to which a cationic group is added. The term "amphoteric galactomannan" refers to a galactomannan polymer to which a cationic group and an anionic group are added such that the polymer has a net positive charge.

Galactomannan polymers are present in the endosperm of seeds of the Leguminosae family. Galactomannan polymers are made up of a combination of mannose monomers and galactose monomers. The galactomannan molecule is a straight chain mannan branched at regular intervals with single membered galactose units on specific mannose units. The mannose units are linked to each other by means of $\beta$ (1-4) glycosidic linkages. The galactose branching arises by way of an $\alpha$ (1-6) linkage. The ratio of mannose monomers to galactose monomers varies according to the species of the plant and also is affected by climate. Non Guar Galactomannan polymer derivatives of the present invention have a ratio of mannose to galactose of greater than 2:1 on a monomer to monomer basis. Suitable ratios of mannose to galactose can be greater than 3:1, and the ratio of mannose to galactose can be greater than 4:1. Analysis of mannose to galactose ratios is well known in the art and is typically based on the measurement of the galactose content.

The gum for use in preparing the non-guar galactomannan polymer derivatives is typically obtained as naturally occurring material such as seeds or beans from plants. Examples of various non-guar galactomannan polymers include but are not limited to Tara gum (3 parts mannose/1 part galactose), Locust bean or Carob (4 parts mannose/1 part galactose), and Cassia gum (5 parts mannose/1 part galactose).

The galactomannan polymer derivative may be a cationic derivative of the non-guar galactomannan polymer, which is obtained by reaction between the hydroxyl groups of the polygalactomannan polymer and reactive quaternary ammonium compounds. Suitable quaternary ammonium compounds for use in forming the cationic galactomannan polymer derivatives include those conforming to the general formulas 1-5, as defined above.

The galactomannan polymer derivative may be an amphoteric galactomannan polymer derivative having a net positive charge, obtained when the cationic galactomannan polymer derivative further comprises an anionic group.

The cationic non-guar galactomannan may have a ratio of mannose to galactose is greater than 4:1. The dispersion compositions of the present invention may comprise a galactomannan polymer derivative by weight of the composition. The shampoo compositions may comprise from 0.05% to 2%, by weight of the composition, of a galactomannan polymer derivative.

(c) Cationically Modified Starch Polymer

The dispersion compositions of the present invention comprise water-soluble cationically modified starch polymers. As used herein, the term "cationically modified starch" refers to a starch to which a cationic group is added prior to degradation of the starch to a smaller molecular weight, or wherein a cationic group is added after modification of the starch to achieve a desired molecular weight. The definition of the term "cationically modified starch" also includes amphoterically modified starch. The term "amphoterically modified starch" refers to a starch hydrolysate to which a cationic group and an anionic group are added.

The dispersion compositions of the present invention comprise cationically modified starch polymers at a range

of 0.01% to 10%, and/or from 0.05% to 5%, by weight of the composition.

The cationically modified starch polymers disclosed herein have a percent of bound nitrogen of from 0.5% to 4% .

As used herein, the term "molecular weight" refers to the weight average molecular weight. The weight average molecular weight may be measured by gel permeation chromatography ("GPC") using a Waters 600E HPLC pump and Waters 717 auto-sampler equipped with a Polymer Laboratories PL Gel MIXED-A GPC column (Part Number 1110-6200, 600.times.7.5 mm, 20 um) at a column temperature of 55.degree. C. and at a flow rate of 1.0 ml/min (mobile phase consisting of Dimethylsulfoxide with 0.1% Lithium Bromide), and using a Wyatt DAWN EOS MALLS (multi-angle laser light scattering detector) and Wyatt Optilab DSP (interferometric refractometer) detectors arranged in series (using a dn/dc of 0.066), all at detector temperatures of 50°C, with a method created by using a Polymer Laboratories narrow dispersed Polysaccharide standard (Mw=47,300), with an injection volume of 200 µl.

The dispersion compositions of the present invention include starch polymers that is chemically modified by the addition of amino and/or ammonium groups into the starch molecules. Non-limiting examples of these ammonium groups may include substituents such as hydroxypropyl trimmonium chloride, trimethylhydroxypropyl ammonium chloride, dimethylstearylhydroxypropyl ammonium chloride, and dimethyldodecylhydroxypropyl ammonium chloride. See Solarek, D. B., Cationic Starches in Modified Starches: Properties and Uses, Wurzburg, O. B., Ed., CRC Press, Inc., Boca Raton, Fla. 1986, pp 113-125. The cationic groups may be added to the starch prior to degradation to a smaller molecular weight or the cationic groups may be added after such modification.

The cationically modified starch polymers of the present invention generally have a degree of substitution of a cationic group from 0.1 to 7. As used herein, the "degree of substitution" of the cationically modified starch polymers is an average measure of the number of hydroxyl groups on each anhydroglucose unit which is derivatized by substituent groups. Since each anhydroglucose unit has three potential hydroxyl groups available for substitution, the maximum possible degree of substitution is 3. The degree of substitution is expressed as the number of moles of substituent groups per mole of anhydroglucose unit, on a molar average basis. The degree of substitution may be determined using proton nuclear magnetic resonance spectroscopy (".sup.1H NMR") methods well known in the art. The source of starch before chemical modification can be chosen from a variety of sources such as tubers, legumes, cereal, and grains. Non-limiting examples of this source starch may include corn starch, wheat starch, rice starch, waxy corn starch, oat starch, cassaya starch, waxy barley, waxy rice starch, glutenous rice starch, sweet rice starch, amioca, potato starch, tapioca starch, oat starch, sago starch, sweet rice, or mixtures thereof.

Cationically modified starch polymers may be selected from degraded cationic maize starch, cationic tapioca, cationic potato starch, and mixtures thereof. Cationically modified starch polymers may be cationic corn starch and cationic tapioca.

The starch, prior to degradation or after modification to a smaller molecular weight, may comprise one or more additional modifications. For example, these modifications may include cross-linking, stabilization reactions, phosphorylations, and hydrolyzations. Stabilization reactions may include alkylation and esterification.

The cationically modified starch polymers in the present invention may be incorporated into the composition in the form of hydrolyzed starch (e.g., acid, enzyme, or alkaline degradation), oxidized starch (e.g., peroxide, peracid, hypochlorite, alkaline, or any other oxidizing agent), physically/mechanically degraded starch (e.g., via the thermomechanical energy input of the processing equipment), or combinations thereof.

An optimal form of the starch is one which is readily soluble in water and forms a substantially clear (% Transmittance.gtoreq.80 at 600 nm) solution in water. The transparency of the composition is measured by Ultra-Violet/Visible (UV/VIS) spectrophotometry, which determines the absorption or transmission of UV/VIS light by a sample, using a Gretag Macbeth Colorimeter Color i 5 according to the related instructions. A light wavelength of 600 nm has been shown to be adequate for characterizing the degree of clarity of cosmetic compositions.

Suitable cationically modified starch for use in compositions of the present invention is available from known starch suppliers. Also suitable for use in the present invention is nonionic modified starch that could be further derivatized to a cationically modified starch as is known in the art. Other suitable modified starch starting materials may be quaternized, as is known in the art, to produce the cationically modified starch polymer suitable for use in the invention.

(d) Cationic cellulose polymers

Suitable cationic cellulose polymers are salts of hydroxyethyl cellulose reacted with trimethyl ammonium substituted epoxide, referred to in the industry (CTFA) as Polyquaternium 10 and available from Dwo/ Amerchol Corp. (Edison, N.J., USA) in their Polymer LR, JR, and KG series of polymers. Other suitable types of cationic cellulose include the polymeric quaternary ammonium salts of hydroxyethyl cellulose reacted with lauryl dimethyl ammonium-substituted epoxide referred to in the industry (CTFA) as Polyquaternium 24. These materials are available from Dow/ Amerchol Corp. under the tradename Polymer LM-200. Other suitable types of cationic cellulose include the polymeric quaternary ammonium salts of hydroxyethyl cellulose reacted with lauryl dimethyl ammonium-substituted epoxide and trimethyl ammonium substituted epoxide referred to in the industry (CTFA) as Polyquaternium 67. These materials are available

from Dow/ Amerchol Corp. under the tradename SoftCAT Polymer SL-5, SoftCAT Polymer SL-30, Polymer SL-60, Polymer SL-100, Polymer SK-L, Polymer SK-M, Polymer SK-MH, and Polymer SK-H.

**D. Mechanical Foam Dispenser**

The hair care composition can be delivered in a liquid or foam form. It may be delivered in a foam form via a mechanical foam dispenser. The mechanical foam dispenser described herein may be selected from the group consisting of squeeze foam dispensers, pump foam dispensers, other mechanical foam dispensers, and combinations thereof. The mechanical foam dispenser may be a squeeze foam dispenser. Non-limiting examples of suitable pump dispensers include those described in WO 2004/078903, WO 2004/078901, and WO 2005/078063 and may be supplied by Albea (60 Electric Ave., Thomaston, CT 06787 USA) or Rieke Packaging Systems (500 West Seventh St., Auburn, Indiana 46706).

The mechanical foam dispenser may comprise a reservoir for holding the hair care composition. The reservoir may be made out of any suitable material selected from the group consisting of plastic, metal, alloy, laminate, and combinations thereof. The reservoir may be a refillable reservoir such as a pour-in or screw-on reservoir, or the reservoir may be for one-time use. The reservoir may also be removable from the mechanical foam dispenser. Alternatively, the reservoir may be integrated with the mechanical foam dispenser. There may be two or more reservoirs.

The reservoir may be comprised of a material selected from the group consisting of rigid materials, flexible materials, and combinations thereof. The reservoir may be comprised of a rigid material if it does not collapse under external atmospheric pressure when it is subject to an interior partial vacuum.

**E. Aerosol Foam Dispenser**

The hair care composition can be delivered in a liquid or foam form. It may be delivered in a foam form via an aerosol foam dispenser. The aerosol foam dispenser may comprise a reservoir for holding the hair care composition. The reservoir may be made out of any suitable material selected from the group consisting of plastic, metal, alloy, laminate, and combinations thereof. The reservoir may be for one-time use. In an embodiment, the reservoir may be removable from the aerosol foam dispenser. Alternatively, the reservoir may be integrated with the aerosol foam dispenser. In an embodiment, there may be two or more reservoirs.

The reservoir may be comprised of a material selected from the group consisting of rigid materials, flexible materials, and combinations thereof. The reservoir may be comprised of a rigid material if it does not collapse under external atmospheric pressure when it is subject to an interior partial vacuum.

**F. Propellant**

The hair care composition described herein may comprise from from 1% to 10% propellant, alternatively from 2% to 9% propellant, and alternatively from 3% to 8% propellant, by weight of the hair care composition.

The propellant may comprise one or more volatile materials, which in a gaseous state, may carry the other components of the hair care composition in particulate or droplet form. The propellant may have a boiling point within the range of from -45° C. to 5° C. The propellant may be liquefied when packaged in convention aerosol containers under pressure. The rapid boiling of the propellant upon leaving the aerosol foam dispenser may aid in the atomization of the other components of the hair care composition.

Aerosol propellant which may be employed in the hair care composition may include the chemically-inert hydrocarbons such as propane, n-butane, isobutane, cyclopropane, and mixtures thereof, as well as halogenated hydrocarbons such as dichlorodifluoromethane, 1,1-dichloro-1,1,2,2-tetrafluoroethane, 1-chloro-1,1-difluoro-2,2-trifluoroethane, 1-chloro-1,1-difluoroethylene, 1,1-difluoroethane, dimethyl ether, monochlorodifluoromethane, trans-1,3,3,3-tetrafluoropropene, and mixtures thereof. The propellant may comprise hydrocarbons such as isobutane, propane, and butane, and these materials may be used for their low ozone reactivity and may be used as individual components where their vapor pressures at 21.1° C. range from 1.17 Bar to 7.45 Bar, alternatively from 1.17 Bar to 4.83 Bar, and alternatively from 2.14 Bar to 3.79 Bar. The propellant may be hydrofluoroolefins (HFOs).

The aerosol foam dispenser may be of the bag on valve type wherein the container comprises an inner bag and an outer container, which encloses the inner bag, while the inner bag has a valve mechanism attached which is movable between an open position and a closed position. The outer container may be formed from metal or plastic or the like, and any of the propellants described herein can be filled in a space between the outer container and the inner bag. The inner bag may be flexible, and can be made from a single material or from a composite material including plastic, which may comprise at least a polymeric layer and a layer which acts as a gas barrier, e.g., made from metal, such as Aluminum. The inner material of the bag may be inert to the contents of the composition, and the inner material may also be impenetrable by the contents of the composition in the bag. The inner bag may comprise a layer of a material

which is essentially impermeable to the propellant inside of the bag. The inner bag may comprise a layer of a material which is essentially impermeable to the propellant outside of the bag which generally is not intended to be mixed with the composition in the inner bag during storage. Where the propellant is inside the bag, it may be known as a foaming agent.

## G. ADDITIONAL COMPONENTS

The shampoo compositions of the present invention may optionally comprise one or more additional components known for use in hair care or personal care products, provided that the additional components are physically and chemically compatible with the essential components described herein, or do not otherwise unduly impair product stability, aesthetics or performance. Such additional components are most typically those described in reference books such as the CTFA Cosmetic Ingredient Handbook, Second Edition, The Cosmetic, Toiletries, and Fragrance Association, Inc. 1988, 1992. Individual concentrations of such additional components may range from 0.001 wt% to 10 wt% by weight of the hair care compositions.

Non-limiting examples of additional components for use in the hair care compositions include conditioning agents, anti-dandruff agents, particles, suspending agents, paraffinic hydrocarbons, propellants, viscosity modifiers, dyes, non-volatile solvents or diluents (water-soluble and water-insoluble), pearlescent aids, foam boosters, additional surfactants or nonionic cosurfactants, pediculocides, pH adjusting agents, perfumes, preservatives, proteins, skin active agents, sunscreens, UV absorbers, and vitamins.

### 1. Conditioning Agent

The hair care compositions may comprise one or more conditioning agents. Conditioning agents include materials that are used to give a particular conditioning benefit to hair. The conditioning agents useful in the hair care compositions of the present invention typically comprise a water-insoluble, water-dispersible, non-volatile, liquid that forms emulsified, liquid particles. Suitable conditioning agents for use in the hair care composition are those conditioning agents characterized generally as silicones, organic conditioning oils or combinations thereof, or those conditioning agents which otherwise form liquid, dispersed particles in the aqueous surfactant matrix. The conditioning agent may contain one or more quaternary ammonium salt in its molecular structure. The conditioning agent may be a dimethiconol

One or more conditioning agents are present from 0.01 wt% to 10 wt%, from 0.1 wt% to 8 wt%, and from 0.2 wt% to 4 wt%, from 0.5 to 1.5% by weight of the composition.

### Silicone Conditioning Agent

The compositions of the present invention may contain one or more silicone conditioning agents. Examples of the silicones include dimethicones, dimethiconols, cyclic silicones, methylphenyl polysiloxane, and modified silicones with various functional groups such as amino groups, quaternary ammonium salt groups, aliphatic groups, alcohol groups, carboxylic acid groups, ether groups, epoxy groups, sugar or polysaccharide groups, fluorine-modified alkyl groups, alkoxy groups, or combinations of such groups. Such silicones may be soluble or insoluble in the aqueous (or non-aqueous) product carrier. In the case of insoluble liquid silicones, the polymer can be in an emulsified form with droplet size of 10 nm to 30 micrometers

Suitable silicone conditioning agents include durable silicone materials such as cross-linkable silicone compounds containing different functional groups including siloxanes or silsequioxanes with terminal hydroxyl or alkoxy function groups. Non-limiting examples include Wacker Belsil ADM 8301E and Belsil ADM 6300E. Other suitable durable conditioning compounds include cross-linkable silicones such as MQ-resin, amino fluids and mixture thereof. Non-limiting examples include Wacker ADM 8500E, Dow Corning DX AP6087, Momentive Silform flexible resins, SR1000 MQ-resin and mxture thereof. Such compounds can cross-link upon drying on hair surface or after exposing to heat treatment to impart durable conditioning over multiple washing cycles.

### Organic Conditioning Materials

The conditioning agent of the compositions of the present invention may also comprise at least one organic conditioning material such as oil or wax, either alone or in combination with other conditioning agents, such as the silicones described above. The organic material can be nonpolymeric, oligomeric or polymeric. It may be in the form of oil or wax and may be added in the formulation neat or in a pre-emulsified form. Some non-limiting examples of organic conditioning materials include, but are not limited to: i) hydrocarbon oils; ii) polyolefins, iii) fatty esters, iv) fluorinated conditioning compounds, v) fatty alcohols, vi) alkyl glucosides and alkyl glucoside derivatives; vii) quaternary ammonium compounds; viii) polyethylene glycols and polypropylene glycols having a molecular weight of up to 2,000,000 including those with CTFA names PEG-20 200, PEG-400, PEG-600, PEG-1000, PEG-2M, PEG-7M, PEG-14M, PEG-45M and

mixtures thereof.

### 2. Rheology Modifier

The hair care product may include one or more rheology modifiers to adjust the rheological characteristics of the composition for better feel, in-use properties and the suspending stability of the composition. For example, the rheological properties are adjusted so that the composition remains uniform during its storage and transportation and it does not drip undesirably onto other areas of the body, clothing or home furnishings during its use. Any suitable rheology modifier can be used. The leave-on treatment may comprise from 0.01% to 3% of a rheology modifier, alternatively from 0.1% to 1% of a rheology modifier.

### 3. Benefit Agents

The hair care composition may further comprise one or more additional benefit agents. The benefit agents comprise a material selected from the group consisting of anti-fungal agents, anti-itch agents, anti-bacterial agents, anti-microbial agents, moisturization agents, anti-oxidants, chelants, vitamins, lipid soluble vitamins, perfumes, brighteners, enzymes, sensates, attractants, dyes, pigments, bleaches, and mixtures thereof.

The shampoo composition may comprise an anti-dandruff active, which may be an anti-dandruff active particulate. The anti-dandruff active can be selected from the group consisting of: pyridinethione salts; azoles, such as an imidazole such as ketoconazole, econazole, climbazole and elubiol; selenium sulphide; coal tar, particulate sulfur; keratolytic agents such as salicylic acid; and mixtures thereof. The anti-dandruff particulate may be a pyridinethione salt.

TEST METHODS

A. Cone/Plate Viscosity Method:

The viscosities of the examples are measured by a Cone/Plate Controlled Stress Brookfield Rheometer R/S Plus, by Brookfield Engineering Laboratories, Stoughton, MA. The cone used (Spindle C-75-1) has a diameter of 75 mm and 1° angle. The viscosity is determined using a steady state flow experiment at constant shear rate of 2 $s^{-1}$ and at temperature of 26.5 °C. The sample size is 2.5ml and the total measurement reading time is 3 minutes. The instrument cannot accurately report the viscosity of a liquid that is below 100cps. The viscosity is reported as less than 100cps.

B. Lather Rheology Method

Lather is generated in a vessel by adding 1) 180 ml of water (having a hardness of 7 grain per gallon) and 2) 20 ml of shampoo. A blade is placed in the center of the vessel. The mixture is blended at a speed of 1200 rpm for 1 minute.

Lather rheology is characterized using an AR2000 Rheometer from TA Instruments. A 60mm acrylate plastic top rotating plate is attached to the rheometer for later rheology measurement. A small amount of lather is applied on the bottom of the plate of the AR 2000 Rheometer using a spatulas. The top plastic plate is lowered with a gap of 1000 microns in between the two plates. The excess lather around the plates is removed. Oscillation stress sweeps are run to generate the elastic modulus or storage modulus (G’), the viscous modulus or loss modulus (G") and Tan Delta (the ratio of G"/G’) of the later. The oscillation frequency is set at 1 Hz, oscillation stress is varied from 0.1 Pa to 25 Pa, and the testing temperature is 25°C. Elastic modulus (G’) of the lather is the average G’ value at oscillation stress range from 0.1 Pa to 0.2 pa.

C. Hair Wet Feel Friction method:

A switch of 4 grams general population hair at 8 inches length is used for the measurement. Water temperature is set at 100°F, (hardness is 7 grain per gallon), and flow rate is 1.6 liter per minute. Amount of 0.4 ml of a liquid shampoo is applied on the hair switch in a zigzag pattern uniformly to cover the entire hair length, using a syringe. The hair switch is then 1$^{st}$ lathered for 30 seconds, rinse with water for 30 seconds, and 2$^{nd}$ lathered for 30 seconds. Water flow rate is then reduced to 0.2 liter per minute. The hair switch is sandwiched with a clamp under 1200 gram of force and pulled through the entire length while the water is running at the low flow rate. The pull time is 30 second. Friction is measured with a friction analyzer (such as Instron or MTS tensile measurement) with a load cell of 5kg. Repeat the pull under rinse for total of 21 times. Total 21 Friction values are collected. Hair Wet Feel Friction ($F_{wet}$) of shampoo is the final rinse friction which is the average friction of the last 7 friction measurements.

D. Hair Clean Rinse Feel method

A switch of 4 grams general population hair at 8 inches length is used for the measurement. Water temperature is set at 100°F, hardness is 7 grain per gallon, and flow rate is 1.6 liter per minute. Amount of 0.4 ml of a liquid shampoo is applied on the hair switch in a zigzag pattern uniformly to cover the entire hair length, using a syringe. The hair switch is then 1$^{st}$ lathered for 30 seconds, rinse with water for 30 seconds, and 2$^{nd}$ lathered for 30 seconds. Water flow rate is then reduced to 0.2 liter per minute. The hair switch is sandwiched with a clamp under 1200 gram of force and pulled through the entire length while the water is running at the low flow rate. The pull time is 30 second. Friction is measured with a Friction analyzer with a load cell of 5kg. Repeat the pull under rinse for total of 21 times. Total 21 Friction values are collected. Hair Wet Feel Friction ($F_{wet}$) of shampoo is the final rinse friction which is the average friction of the last 7 friction measurements. Then, the water is shut off.

The hair switch is still sandwiched with the clamp under 1200 gram of force and pulled through the entire length. The pull time is 30 second. Friction is measured with the Friction analyzer with the load cell of 5kg. Repeat the pull for total of 10 times. Hair Rinse Feel Friction ($F_{rinse}$) of shampoo is the final rinse friction which is the average friction of the last 5 friction measurements. Hair Clean Rinse Feel ($F_{clean}$) is the magnitude of friction reduction from Hair Wet Feel Friction ($F_{wet}$) to Hair Rinse Feel Friction ($F_{rinse}$).

$$F_{clean} = F_{wet} - F_{rinse}$$

E. Hair Switch Soil Removal Method

A switch of 4 grams general population hair at 8 inches length is used for the measurement. It's weighted and recorded as WO. Amount of 2ml of coconut oil (Crodamol GTCC-LQ-(MV), supplied from Croda) is applied on the hair switch in a zigzag pattern uniformly to cover the entire hair length, using a syringe. The hair switch is then rubbed for 30 second to soak all of the oil into the hair switch. The soiled hair switch is weighted and recorded as W1. The Soil applied on the hair switch is calculated as W1-WO and recorded as Wsoil applied. Water is then turned on. The temperature is set at 100oF, hardness is 7 grain per gallon, and flow rate is 1.6 liter per minute. The soiled hair switch is wetted with water for 1 second. Amount of 0.4 ml of a liquid shampoo is applied on the hair switch in a zigzag pattern uniformly to cover the entire hair length, using a syringe. The hair switch is lathered for 1 minute, rinsed with water for 1 minute, and squeezed to let the excess water out. The hair switch is hung on a rack and dried overnight. The cleaned and air dried hair switch is weighted and recorded as W2. The weight of oil residue left on the hair switch is calculated W2-WO and recorded as Wsoil residue.

The Weight Percent Soil Residue left on the hair switch is calculated as

$$\text{\% Soil Residue left on hair switch} = W_{soil\ residue} / W_{soil\ applied}\ x100$$

The Weight Percent of Soil Removed from hair switch is calculated as:

$$\text{\% Soil Removed from hair switch} = (W_{soil\ applied} - W_{soil\ residue}) / W_{soil\ applied}\ x100$$

F. Hair Switch Expansion Method

A switch of 4 grams general population hair at 8 inches length is used for the measurement. It's weighted and recorded as WO. Amount of 2ml of coconut oil (Crodamol GTCC-LQ-(MV), supplied from Croda) is applied on the hair switch in a zigzag pattern uniformly to cover the entire hair length, using a syringe. The hair switch is then rubbed for 30 second to soak all of the oil into the hair switch. The soiled hair switch is then hung in front of a lighted white board with back lights from behind. Pictures are then taken. The images are analyzed using Image Pro 7 Analyzer. Bulk area of the soiled hair switch is reported as Asoiled in number of pixels. Water is then turned on. The temperature is set at 100°F, hardness is 7 grain per gallon, and flow rate is 1.6 liter per minute. The soiled hair switch is wetted with water for 1 second. Amount of 0.4 ml of a liquid shampoo is applied on the hair switch in a zigzag pattern uniformly to cover the entire hair length, using a syringe. The hair switch is lathered for 1 minute, rinsed with water for 1 minute, and squeezed to let the excess water out. The hair switch is hung on a rack and dried overnight. The cleaned and air dried hair switch is hung in front of the lighted white board with back lights from behind. Pictures are then taken. The images are analyzed using Image Pro 7 Analyzer. Bulk area of the soiled hair switch is reported as Acleaned in number of pixels.

The Area expansion of hair switch is calculated as:

$$\text{Area Expansion of Hair Switch} = A_{cleaned} - A_{soiled}$$

The Percent expansion of hair switch is calculated as:

$$\text{\% Expansion of Hair Switch} = (A_{cleaned} - A_{soiled}) / D_{soiled}\ x100$$

G. Hair Flexibility Method

A switch of 4 grams general population hair at 8 inches length is used for the measurement. Water temperature is set at 100°F, hardness is 7 grain per gallon, and flow rate is 1.6 liter per minute. An amount of 0.4 ml of a shampoo is applied on the hair switch in a zigzag pattern uniformly to cover the entire hair length using a syringe. The hair switch is lathered for 1 minute, rinsed with water for 1 minute, and squeezed to let the excess water out. The hair switch is hung on a rack and dried overnight. Three replicates of hair switches are washed and dried for each shampoo.

Hair Flexibility of the dried hair switches is assessed by six panelists. Panelists are asked to grade the hair switches on a 0 to 10 scale (0 = low, 10 = high). Panelists' responses are then averaged to assign a hair flexibility score for the shampoo example.

## EXAMPLES

METHOD OF MAKING

The following examples illustrate embodiments of the invention described herein. The exemplified hair care compositions may be made by conventional formulation and mixing techniques or by mixing together one or more polyols (e.g. glycerin, propylene glycol etc.), water and surfactants along with any solids that need to be melted at an elevated temperature, e.g. 75°C. The ingredients are mixed thoroughly at the elevated temperature and then cooled to ambient temperature. Additional ingredients, including electrolytes, polymers, silicone emulsions, preservatives and fragrances may be added to the cooled product. All parts, percentages, and ratios herein are by weight unless otherwise specified. Some components may come from suppliers as dilute solutions. The amount stated reflects the weight percent of the active material, unless otherwise specified.

The following are non-limiting examples of Hair Care compositions described herein. They do not fall within the scope of claim 1 of the present application.

Table 1 Cleansing shampoo compositions

| | Comp. Ex. 1 | Comp. Ex. 2 | Ex. 1 | Ex. 2 | Ex. 3 |
|---|---|---|---|---|---|
| Appearance | Clear, stable | Clear, stable | Clear, stable | Clear, stable | Clear, stable |
| Stability | Yes | Yes | Yes | Yes | Yes |
| Viscosity (cP) | 5 | 6 | 16 | 43 | 321 |
| Glycerin | 0 | 10 | 30 | 50 | 70 |
| Water | 83.8 | 73.8 | 53.8 | 33.8 | 13.8 |
| Sodium Laureth Sulfate (SLE1S-70% active) [1] | 8 | 8 | 8 | 8 | 8 |
| Sodium Tridecyl Ether Sulfate (ST2S-65 active)[10] | 7 | 7 | 7 | 7 | 7 |
| pH adjusters to adjust pH 5.7 ± 0.3 | | | | | |
| Glycerin/water ratio | 0.00 | 0.14 | 0.56 | 1.48 | 5.07 |
| Total Detersive Surfactant | 15 | 15 | 15 | 15 | 15 |
| % Soil Residual left | 5.05% | 3.64% | 1.96% | 2.42% | 1.77% |
| Example 1 is not within the scope of the present invention. | | | | | |

Table 2 Cleansing shampoo compositions

| | Ex. 4 | Ex. 5 | Ex. 6 | Ex. 7 | Ex. 8 | Ex. 9 | Ex. 10 |
|---|---|---|---|---|---|---|---|
| Appearance | Clear, stable | Clear, stable | Clear, stable | Clear, stable | Clear, stable | Clear, stable | Clear, stable |
| Stability | Yes | Yes | Yes | Yes | Yes | Yes | Yes |
| Viscosity (cP) | 212 | 69 | 6466 | 2114 | 735 | 162 | 790 |
| Glycerin | 80 | 70 | 50 | 70 | 72 | 50 | 68.9 |
| Water | 10.8 | 20.8 | 35.8 | 15.8 | 8.81 | 28.8 | 9.89 |
| Sodium Laureth Sulfate (SLE1S-70% active) [1] | 8 | 8 | 8 | 8 | 8 | 8 | 8 |
| Sodium Tridecyl Ether Sulfate (ST2S-65 active)[10] | | | 5 | 5 | 10 | 12 | 12 |
| pH adjusters to adjust pH 5.7 ± 0.3 | | | | | | | |
| Glycerin/water ratio | 7.41 | 3.37 | 1.40 | 4.43 | 8.17 | 1.74 | 6.97 |
| Total Detersive Surfactant | 8 | 8 | 13 | 13 | 18 | 20 | 20 |

Table 3 Cleansing shampoo compositions

| | Ex. 11 | Ex. 12 | Ex. 13 | Ex. 14 | Ex. 15 | Ex. 16 |
|---|---|---|---|---|---|---|
| Appearance | Clear, stable | Clear, stable | Clear, stable | Clear, stable | Clear, stable | Clear, stable |
| Stability | Yes | Yes | Yes | Yes | Yes | Yes |
| Viscosity (cP) | 1410 | 903 | 782 | 1051 | 1039 | 1542 |
| Glycerin | 53.53 | 30 | 46.7 | 46.7 | 46.7 | 46.7 |
| Water | 15.27 | 38.8 | 23.3 | 22.7 | 23.3 | 22.7 |
| Sodium Laureth Sulfate (SLE1S-70% active) [1] | 8 | 8 | | | | |
| Sodium Tridecyl Ether Sulfate (ST2S-65 active)[10] | 22 | 22 | 26 | 26 | | |
| Sodium Tridecyl Ether Sulfate (ST3S-65 active)[11] | | | | | 26 | 26 |
| Cocoamdopropyl Betaine (CAPB 30% active)[4] | | | 4 | | 4 | |
| LAPB (35%)[7] | | | | 4.7 | | 4.7 |
| pH adjusters to adjust pH 5.7 ± 0.3 | | | | | | |
| Glycerin/water ratio | 3.51 | 0.77 | 2 | 2.1 | 2 | 2.1 |
| Total Detersive Surfactant | 30 | 30 | 30 | 31 | 30 | 31 |
| Example 12 is not within the scope of the present invention. | | | | | | |

Table 4 Cleansing shampoo compositions

| | Ex. 17 | Ex. 18 | Ex. 19 | Ex. 20 | Ex. 21 | Ex. 22 |
|---|---|---|---|---|---|---|
| Glycerin | 30 | 50 | 70 | - | - | - |
| Propylene Glycol | - | - | - | 30 | 50 | 70 |
| Water | 53.8 | 33.8 | 13.8 | 53.8 | 33.8 | 13.8 |
| Sodium Laureth Sulfate (SLE1S-70% active) [1] | 5 | 5 | 5 | 5 | 5 | 5 |
| Sodium Tridecyl Ether Sulfate (ST2S-65 active)[10] | 10 | 10 | 10 | 10 | 10 | 10 |
| Perfume | 1.2 | 1.2 | 1.2 | 1.2 | 1.2 | 1.2 |
| Glycerin/water ratio | 0.56 | 1.48 | 5.07 | - | - | - |
| Propylene Glycol/water ratio | - | - | - | 0.56 | 1.48 | 5.07 |
| Total Detersive Surfactant | 15 | 15 | 15 | 15 | 15 | 15 |
| pH adjusters to adjust pH 5.7 ± 0.3 | | | | | | |
| Examples 17 and 20 are not within the scope of the present invention. | | | | | | |

Table 5 Cleansing shampoo compositions

| | Ex. 23 | Ex. 24 | Ex. 25 | Ex. 26 | Ex. 27 | Ex. 28 |
|---|---|---|---|---|---|---|
| Glycerin | 50 | 50 | 50 | 50 | 70 | 70 |
| Water | 33.7 | 33.2 | 33.1 | 32.1 | 13.6 | 13.6 |
| Sodium Laureth Sulfate (SLE1S-70% active) [1] | 5 | 5 | 5 | 5 | 5 | 5 |
| Sodium Tridecyl Ether Sulfate (ST2S-65 active)[10] | 10 | 10 | 10 | 10 | 10 | 10 |
| Guar, Hydroxylpropyl Trimonium Chloride, Jaguar C-500[3] | 0.1 | 0.1 | 0.2 | 0.2 | - | - |
| Silicone Quaternium [4] | - | 0.5 | 0.5 | 1.5 | - | - |
| Polyquaternium-10, Ucare LR400[5] | - | - | - | - | 0.2 | - |
| Polyquaternium-67, Softcat SX1300[6] | - | - | - | - | - | 0.2 |
| Perfume | 1.2 | 1.2 | 1.2 | 1.2 | 1.2 | 1.2 |
| Glycerin/water ratio | 1.48 | 1.51 | 1.51 | 1.56 | 5.15 | 5.15 |
| Total Detersive Surfactant | 15 | 15 | 15 | 15 | 15 | 15 |
| pH adjusters to adjust pH 5.7 ± 0.3 | | | | | | |

Table 6: Cleansing shampoo compositions

| | Ex. 29 | Ex. 30 | Ex. 31 |
|---|---|---|---|
| Glycerin | 71.9 | 69.5 | 68.1 |
| Water | 13.1 | 14.5 | 14.9 |
| Sodium Laureth Sulfate (SLE1S-70% active) [1] | 3 | 4 | 4 |
| Sodium Tridecyl Ether Sulfate (ST2S-65 active)[10] | 12 | 12 | 13 |
| Glycerin/water ratio | 5.5 | 4.8 | 4.6 |
| Total Detersive Surfactant | 15 | 16 | 17 |
| pH adjusters to adjust pH 5.7 ± 0.3 | | | |

Table 7 Cleansing shampoo compositions

| | Ex. 32 | Ex. 33 |
|---|---|---|
| Glycerin | 71.4 | 73.6 |
| Water | 12.6 | 11.4 |
| Lauramidopropyl Betaine (LAPB 35% active)[7] | 4 | 3.5 |
| Sodium Tridecyl Ether Sulfate (ST2S-65 active)[10] | 12 | 11.5 |
| Glycerin/water ratio | 5.7 | 6.4 |
| Total Detersive Surfactant | 16 | 15 |
| pH adjusters to adjust pH 5.7 ± 0.3 | | |

Table 8 Cleansing shampoo compositions

| | Ex 34 | Ex 35 | Ex 36 | Ex 37 |
|---|---|---|---|---|
| Glycerin | 42.9 | 36.2 | 45.7 | 39.0 |
| Water | 25.1 | 29.8 | 24.3 | 29.0 |
| Sodium Laureth Sulfate (SLE1S-70% active) [1] | 6 | 8 | 6 | 8 |
| Sodium Tridecyl Ether Sulfate (ST2S-65 active)[10] | 26 | 26 | 24 | 24 |
| Glycerin/water ratio | 1.7 | 1.2 | 1.9 | 1.3 |
| Total Detersive Surfactant | 32 | 34 | 30 | 32 |
| pH adjusters to adjust pH 5.7 ± 0.3 | | | | |
| Example 37 is not within the scope of the present invention. | | | | |

Table 9 Cleansing shampoo compositions

| | Ex 38 | Ex 39 | Ex 40 | Ex 41 | Ex 42 |
|---|---|---|---|---|---|
| Glycerin | 39.5 | 46.7 | 46.7 | 46.7 | 46.7 |
| Water | 26.5 | 23.3 | 22.7 | 23.3 | 22.7 |
| Sodium Laureth Sulfate (SLE3 S-70% active)[9] | 5 | - | - | - | - |
| Sodium Tridecyl Ether Sulfate (ST2S-65 active)[10] | 20 | 26 | 26 | - | - |
| Sodium Tridecyl Ether Sulfate (ST3S-65 active)[11] | - | - | - | 26 | 26 |
| Cocoamdopropyl Betaine (CAPB 30% active)[2] | 4 | 4 | - | 4 | - |
| LAPB (35%)[7] | - | - | 4.7 | - | 4.7 |
| Lauryl Hydroxysultaine (LHS 42.5% active)[12] | 4 | - | - | - | - |
| Glycerin/water ratio | 1.5 | 2.0 | 2.1 | 2.0 | 2.1 |

(continued)

| | Ex 38 | Ex 39 | Ex 40 | Ex 41 | Ex 42 |
|---|---|---|---|---|---|
| Total Detersive Surfactant | 34 | 30 | 31 | 30 | 31 |

Example 38 is not within the scope of the present invention.
1. Sodium Laureth (1 molar ethylene oxide) sulfate at 70% active, supplier: Stephan Co
2. Tegobetaine F-B, 30% active, supplier: Goldschmidt Chemical
3. Jaguar C500, MW of 500,000, CD of 0.8, from Rhodia
4. Silicone quaternium micro-emulsion, 30% active, Abil ME 45, from Evonik
5. Polyquaternium-10, Ucare LR400, from Dow Chemical
6. Polyquaternium-67, Softcat SX1300, from Dow Chemical
7. LAPB (Mackam DAB), at 35% active level, supplier: Rhodia
8. LAPB (Mackam 1200), at 84% active level, supplier: Rhodia
9. Sodium Laureth (3 molar ethylene oxide) sulfate at 70% active, supplier: Stephan Co
10. Sodium Tridecyl Ether Sulfate (2 molar ethylene oxide), Stepan ST2S-65 (Steol-TD 402 65) 65% active, supplier: Stephan Co
11. Sodium Tridecyl Ether Sulfate (3 molar ethylene oxide), Stepan ST3S-65 (Steol-TD 403 65) 65% active, supplier: Stephan Co
12. LHS (Mackam LHS) at 42.5% active level, supplier: Rhodia

**FOAMED COMPOSITION EXAMPLES**

The hair care composition can be delivered in a liquid or foam form. It may be delivered in a foam form via a mechanical foam dispenser when the hair care composition has a viscosity less than 500 cps. It may be further delivered in a foam form via an aerosol foam dispenser with 1 to 10 weight percent of a propellant when the hair care composition has a viscosity less than 3000 cps. The viscosity values are measured before adding in the propellants.

Table 10 Cleansing foam shampoo composition via Mechanical Pump Foamer

| | Ex. A | Ex. B | Ex. C | Ex. D | Ex. E | Ex. F |
|---|---|---|---|---|---|---|
| Appearance | Clear , stable | Clear , stable | Clear , stable | Clear , stable | Clear , stable | Clear , stable |
| Stability | Yes | Yes | Yes | Yes | Yes | Yes |
| Viscosity (cP) | 16 | 43 | 321 | 212 | 69 | 162 |
| Glycerin | 30 | 50 | 70 | 80 | 70 | 50 |
| Water | 53.8 | 33.8 | 13.8 | 10.8 | 20.8 | 28.8 |
| Sodium Laureth Sulfate (SLE1S-70% active) | 8 | 8 | 8 | 8 | 8 | 8 |
| Sodium Tridecyl Ether Sulfate (ST2S-65 active)[10] | 7 | 7 | 7 | | | 12 |
| Glycerin/water ratio | 0.56 | 1.48 | 5.07 | 7.41 | 3.37 | 1.74 |
| Total Detersive Surfactant | 15 | 15 | 15 | 8 | 8 | 20 |

Example A is not within the scope of the present invention.

Table 13 Cleansing foam shampoo composition via Aerosol Foam Former

| | Ex. G | Ex. H | Ex. I | Ex. J | Ex. K | Ex. L | Ex. M | Ex. N | Ex. O |
|---|---|---|---|---|---|---|---|---|---|
| Appearance | Clear, stable | Clear, stable | Clear, stable | Clear, stable | Clear, stable | Clear, stable | Clear, stable | Clear, stable | Clear, stable |

(continued)

| | Ex. G | Ex. H | Ex. I | Ex. J | Ex. K | Ex. L | Ex. M | Ex. N | Ex. O |
|---|---|---|---|---|---|---|---|---|---|
| Stability | Yes | Yes | Yes | Yes | Yes | Yes | Yes | Yes | Yes |
| Bulk Viscosity (cps) | 1410 | 903 | 782 | 1051 | 1039 | 1542 | 735 | 162 | 790 |
| Glycerin | 53.53 | 30 | 46.7 | 46.7 | 46.7 | 46.7 | 72 | 50 | 68.9 |
| Water | 15.27 | 38.8 | 23.3 | 22.7 | 23.3 | 22.7 | 8.81 | 28.8 | 9.89 |
| Sodium Laureth Sulfate (SLE1S-70% active) | 8 | 8 | | | | | 8 | 8 | 8 |
| Sodium Tridecyl Ether Sulfate (ST2S-65 active)[10] | 22 | 22 | 26 | 26 | | | 10 | 12 | 12 |
| Sodium Tridecyl Ether Sulfate (ST3S-65 active)[11] | | | | | 26 | 26 | | | |
| Cocoamdopropyl Betaine (CAPB 30% active)[2] | | | 4 | | 4 | | | | |
| LAPB (35%)[7] | | | | 4.7 | | 4.7 | | | |
| Propellant HFO [16] | 5.5 | 5.5 | 5.5 | 5.5 | 5.5 | 5.5 | 5.5 | 5.5 | 5.5 |
| Glycerin/water ratio | 3.51 | 0.77 | 2 | 2.1 | 2 | 2.1 | 8.17 | 1.74 | 6.97 |
| Total Detersive Surfactant | 30 | 30 | 30 | 31 | 30 | 31 | 18 | 20 | 20 |
| pH adjusters to adjust pH 5.7 ± 0.3 | | | | | | | | | |
| Example H is not within the scope of the present invention. | | | | | | | | | |

Table 14 Cleansing foam shampoo composition via Aerosol Foam Former

| | Ex. P | Ex. Q | Ex. R | Ex. S | Ex. T | Ex. U | Ex. V | Ex. W | Ex. X |
|---|---|---|---|---|---|---|---|---|---|
| Appearance | Clear, stable | Clear, stable | Clear, stable | Clear, stable | Clear, stable | Clear, stable | Clear , stabl e | Clear, stable | Clear, stable |
| Stability | Yes | Yes | Yes | Yes | Yes | Yes | Yes | Yes | Yes |
| Bulk Viscosity (cps) | 1410 | 903 | 782 | 1051 | 1039 | 1542 | 735 | 162 | 790 |
| Glycerin | 53.53 | 30 | 46.7 | 46.7 | 46.7 | 46.7 | 72 | 50 | 68.9 |
| Water | 15.27 | 38.8 | 23.3 | 22.7 | 23.3 | 22.7 | 8.81 | 28.8 | 9.89 |
| Sodium Laureth Sulfate (SLE1S-70% active) | 8 | 8 | | | | | 8 | 8 | 8 |
| Sodium Tridecyl Ether Sulfate (ST2S-65 active)[10] | 22 | 22 | 26 | 26 | | | 10 | 12 | 12 |
| Sodium Tridecyl Ether Sulfate (ST3S-65 active)[11] | | | | | 26 | 26 | | | |

(continued)

| | Ex. P | Ex. Q | Ex. R | Ex. S | Ex. T | Ex. U | Ex. V | Ex. W | Ex. X |
|---|---|---|---|---|---|---|---|---|---|
| Cocoamdopropyl Betaine (CAPB 30% active)[2] | | | 4 | | 4 | | | | |
| LAPB (35%)[7] | | | | 4.7 | | 4.7 | | | |
| Propellant A46 [15] | 4 | 4 | 4 | 4 | 4 | 4 | 4 | 4 | 4 |
| Glycerin/water ratio | 3.51 | 0.77 | 2 | 2.1 | 2 | 2.1 | 8.17 | 1.74 | 6.97 |
| Total Detersive Surfactant | 30 | 30 | 30 | 31 | 30 | 31 | 18 | 20 | 20 |
| pH adjusters to adjust pH 5.7 ± 0.3 | | | | | | | | | |
| Example Q is not within the scope of the present invention.<br>15. Aeron A-Blends, A46 (Isobutane/Propane = 84.85/15.15) from Diversified CPC International<br>16. Hydrofluoroolefins (HFO-1234ze) from Honeywell | | | | | | | | | |

**Claims**

**1.** A hair care composition comprising:

a) from 5 to 40 wt.% of the hair care composition of one or more detersive surfactants wherein less than 10 wt.% of the hair care composition comprises a linear anionic surfactant selected from the group consisting of:

(1) alkyl sulfates

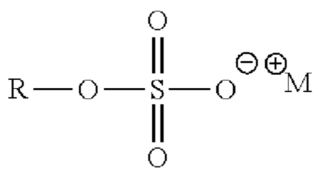

Alkyl sulfates

where R is a linear $C_8$-$C_{24}$ alkyl and $M^+$ is monovalent cation;
(2) alkyl ether sulfates

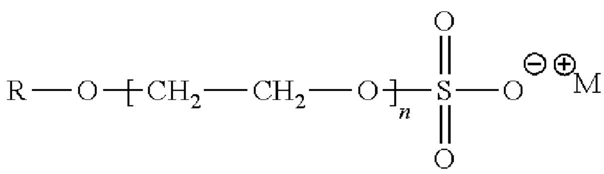

Alkyl ether sulfates

where R is a linear $C_8$-$C_{24}$ alkyl, n=1-2, and $M^+$ is monovalent cation;
(3) and mixtures thereof,

wherein one of the detersive surfactant is a branched anionic surfactant selected from the group consisting of:

(1) alkyl sulfates

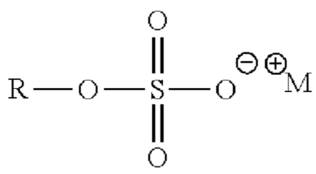

Alkyl sulfates

where R is a branched $C_8$-$C_{24}$ alkyl and $M^+$ is monovalent cation;
(2) alkyl ether sulfates

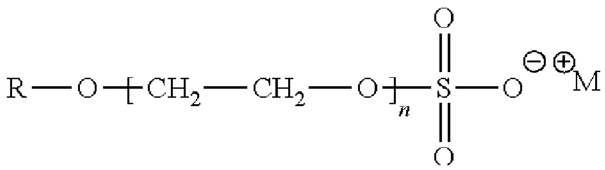

Alkyl ether sulfates

where R is a branched $C_8$-$C_{24}$ alkyl, n=1-2, and $M^+$ is monovalent cation;
and mixtures thereof;

b) a carrier comprising one or more polyols and water, wherein the carrier comprises from 40 to 70 wt.% of the hair care composition of one or more polyols, and from 9% to 75% by weight of the hair care composition of water; and wherein the weight ratio of one or more polyols to water is 0.4 or higher.

**2.** The hair care composition according to claim 1, wherein one of the detersive surfactant is selected from the group consisting of:

i) alkyl betaines

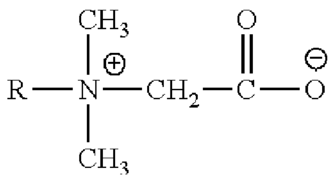

Alkyl betaines

where R is $C_8$-$C_{24}$ alkyl, saturated or unsaturated, or mixtures thereof;
ii) alkyl hydroxysultaines

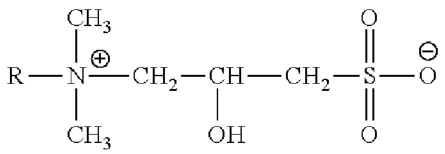

Alkyl hydroxysultaines

where R is $C_8$-$C_{24}$ alkyl, saturated or unsaturated, or mixtures thereof;
iii) alkyl amhoacetates

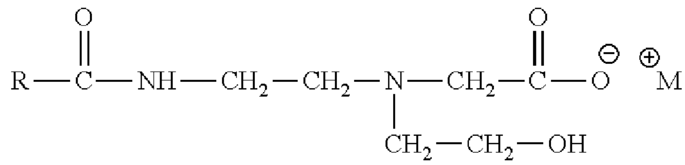

Alkyl amphoacetates

where R is $C_6$-$C_{24}$ alkyl, saturated or unsaturated, or mixtures thereof and $M^+$ is monovalent cation; and
iv) mixtures thereof.

3. The hair care composition according to any of the preceding claims, wherein the one or more polyols is selected from the group consisting of glycerin, ethylene glycol, propylene glycol, di-propylene glycol, ethylene glycol and mixtures thereof, preferably wherein the one or more polyol is glycerin.

4. The hair care composition according to any of the preceding claims, comprising from 10 to 28 wt.% of the hair care composition of an anionic surfactant, preferably from 10 to 20 wt.% of the hair care composition of an anionic surfactant, even more preferably from 10 to 15 wt.% of the hair care composition of an anionic surfactant.

5. The hair care composition according to any of the preceding claims, comprising from 2% to 15 wt.% of the hair care composition of zwitterionic surfactant.

6. The hair care composition according to any of the preceding claims, wherein the detersive surfactant is one of more nonionic surfactants.

7. The hair care composition according to any of the preceding claims, wherein the hair care composition further contains 0.05 to 5 wt.% of a silicone conditioning agent, preferably wherein the silicone conditioning agent contains one of more quaternary ammonium salt in its molecular structure, even more preferably wherein the silicone conditioning agent is dimethiconol

8. The hair care composition according to any of the preceding claims, wherein the hair care composition further comprises from 0.05 to 2 wt.% of the hair care composition of one or more cationic polymers, preferably wherein the cationic polymers are selected from the group consisting of guar hydroxylpropyltrimonium chloride, Polyquaternium-6, Polyquaternium-7, Polyquaternium-10, Polyquaternium-39, Polyquaterinum-67, and mixtures thereof.

9. The hair care composition according to claim 8, wherein the guar hydroxylpropyltrimonium chloride has a weight average molecular weight of from 50,000 to 1.0 million g/mol, preferably wherein the guar hydroxylpropyltrimonium chloride has a weight average molecular weight of from 100,000 to 900,000 g/mol.

**Patentansprüche**

1. Haarpflegezusammensetzung, umfassend:

a) zu 5 bis 40 Gew.-% der Haarpflegezusammensetzung ein oder mehrere Reinigungstenside, wobei weniger als 10 Gew.-% der Haarpflegezusammensetzung ein lineares anionisches Tensid umfassen, ausgewählt aus der Gruppe bestehend aus:

(1) Alkylsulfaten

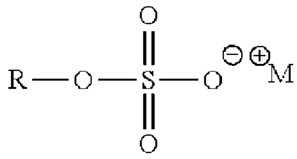

Alkylsulfate

wobei R ein lineares $C_8$-$C_{24}$-Alkyl ist und $M^+$ ein einwertiges Kation ist;

(2) Alkylethersulfaten

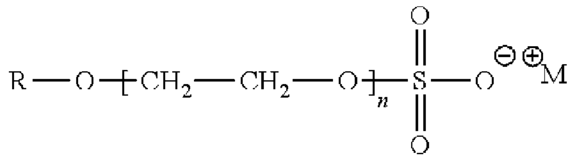

Alkylethersulfate

wobei R ein lineares $C_8$-$C_{24}$ -Alkyl ist, n=1-2, und $M^+$ ein einwertiges Kation ist;

(3) und Mischungen davon,

wobei eines des Reinigungstensids ein verzweigtes anionisches Tensid ist, ausgewählt ist aus der Gruppe bestehend aus:

(1) Alkylsulfaten

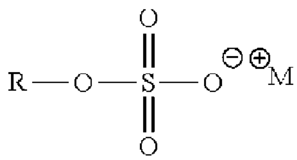

Alkylsulfate

wobei R ein verzweigtes $C_8$-$C_{24}$-Alkyl ist und $M^+$ ein einwertiges Kation ist;

(2) Alkylethersulfaten

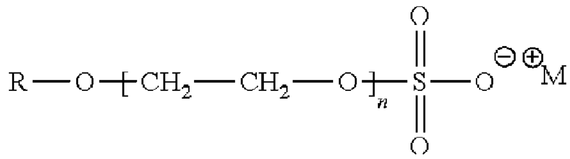

Alkylethersulfate

wobei R ein verzweigtes $C_8$-$C_{24}$ -Alkyl ist, n=1-2, und $M^+$ ein einwertiges Kation ist;

und Mischungen davon;

b) einen Träger, umfassend ein oder mehrere Polyole und Wasser, wobei der Träger zu 40 bis 70 Gew.-% der Haarpflegezusammensetzung ein oder mehrere Polyole und zu 9 Gew.-% bis 75 Gew.-% der Haarpflegezusammensetzung Wasser umfasst; und wobei das Gewichtsverhältnis von einem oder mehreren Polyolen zu Wasser 0,4 oder höher beträgt.

2. Haarpflegezusammensetzung nach Anspruch 1, wobei eines des Reinigungstensids ausgewählt ist aus der Gruppe bestehend aus:

i) Alkylbetainen

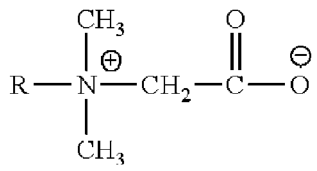

Alkylbetaine

wobei R $C_8$-$C_{24}$ -Alkyl, gesättigt oder ungesättigt, oder Mischungen davon ist;
ii) Alkylhydroxysultainen

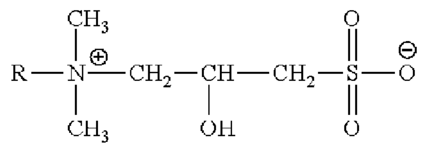

Alkylhydroxysultaine

wobei R $C_8$-$C_{24}$ -Alkyl, gesättigt oder ungesättigt, oder Mischungen davon ist;
iii) Alkylamphoacetaten

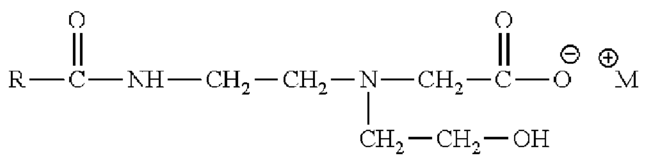

Alkylamphoacetate

wobei R $C_6$-$C_{24}$ -Alkyl, gesättigt oder ungesättigt, oder Mischungen davon ist und $M^+$ ein einwertiges Kation ist; und
iv) Mischungen davon.

3. Haarpflegezusammensetzung nach einem der vorstehenden Ansprüche, wobei das eine oder die mehreren Polyole ausgewählt sind aus der Gruppe bestehend aus Glycerin, Ethylenglykol, Propylenglykol, Dipropylenglykol, Ethylenglykol und Mischungen davon, wobei das eine oder die mehreren Polyole Glycerin sind.

4. Haarpflegezusammensetzung nach einem der vorstehenden Ansprüche, umfassend zu 10 bis 28 Gew.-% der Haarpflegezusammensetzung ein anionisches Tensid, vorzugsweise von 10 bis 20 Gew.-% der Haarpflegezusammensetzung ein anionisches Tensid, noch mehr bevorzugt von 10 bis 15 Gew.-% der Haarpflegezusammensetzung ein anionisches Tensid.

5. Haarpflegezusammensetzung nach einem der vorstehenden Ansprüche, umfassend zu 2 % bis 15 Gew.-% der Haarpflegezusammensetzung ein zwitterionisches Tensid.

6. Haarpflegezusammensetzung nach einem der vorstehenden Ansprüche, wobei das Reinigungstensid eines von mehreren nichtionischen Tensiden ist.

**7.** Haarpflegezusammensetzung nach einem der vorstehenden Ansprüche, wobei die Haarpflegezusammensetzung ferner zu 0,05 bis 5 Gew.-% ein Silikon-Konditioniermittel enthält, vorzugsweise wobei das Silikon-Konditioniermittel eines von mehreren quaternären Ammoniumsalzen in seiner Molekülstruktur enthält, noch mehr bevorzugt wobei das Silikon-Konditioniermittel Dimethiconol ist.

**8.** Haarpflegezusammensetzung nach einem der vorstehenden Ansprüche, wobei die Haarpflegezusammensetzung ferner zu 0,05 bis 2 Gew.-% der Haarpflegezusammensetzung ein oder mehrere kationische Polymere umfasst, vorzugsweise wobei die kationischen Polymere ausgewählt sind aus der Gruppe bestehend aus Guargummihydroxylpropyltrimoniumchlorid, Polyquaternium-6, Polyquaternium-7, Polyquaternium-10, Polyquaternium-39, Polyquaternium-67 und Mischungen davon.

**9.** Haarpflegezusammensetzung nach Anspruch 8, wobei das Guargummihydroxylpropyltrimoniumchlorid ein durchschnittliches Molekulargewicht von 50.000 bis 1,0 Million g/mol aufweist, vorzugsweise wobei das Guargummihydroxylpropyltrimoniumchlorid ein durchschnittliches Molekulargewicht von 100.000 bis 900.000 g/mol aufweist.

## Revendications

**1.** Composition pour soins capillaires comprenant :

a) de 5 à 40 % en poids de la composition pour soins capillaires d'un ou plusieurs agents tensioactifs détersifs dans laquelle moins de 10 % en poids de la composition pour soins capillaires comprennent un agent tensioactif anionique linéaire choisi dans le groupe constitué de :

(1) sulfates d'alkyle

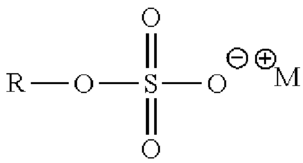

1

Sulfates d'alkyle
où R est un alkyle en $C_8$ à $C_{24}$, et $M^+$ est un cation monovalent ;
(2) Sulfates d'alkyléther

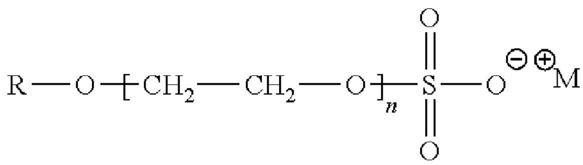

Sulfates d'alkyléther
où R est un alkyle linéaire en $C_8$ à $C_{24}$, n=1-2, et $M^+$ est un cation monovalent ;
(3) et des mélanges de ceux-ci,

dans laquelle l'agent tensioactif est un agent tensioactif anionique ramifié choisi dans le groupe constitué de :

(1) sulfates d'alkyle

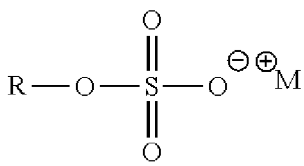

Sulfates d'alkyle
où R est un alkyle ramifié en $C_8$ à $C_{24}$, et $M^+$ est un cation monovalent ;
(2) Sulfates d'alkyléther

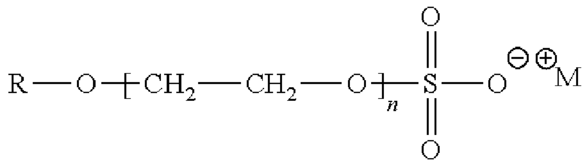

Sulfates d'alkyléther

où R est un alkyle ramifié en $C_8$ à $C_{24}$, n=1-2, et $M^+$ est un cation monovalent ;
et des mélanges de ceux-ci ;

b) un support comprenant un ou plusieurs polyols et de l'eau, dans laquelle le support comprend de 40 à 70 % en poids de la composition pour soins capillaires d'un ou plusieurs polyols, et de 9 % à 75 % en poids de la composition pour soins capillaires d'eau ; et dans laquelle le rapport en poids d'un ou plusieurs polyols à l'eau est de 0,4 ou plus.

**2.** Composition pour soins capillaires selon la revendication 1, dans laquelle l'agent tensioactif détersif est choisi dans le groupe constitué de :

i) Alkyl bétaïnes

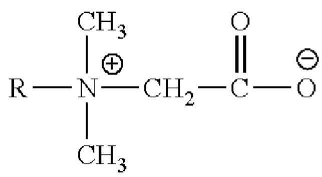

Alkyl bétaïnes
où R est un alkyle en $C_8$ à $C_{24}$, saturé ou insaturé, ou des mélanges de ceux-ci ;
ii) Hydroxysultaines d'alkyle

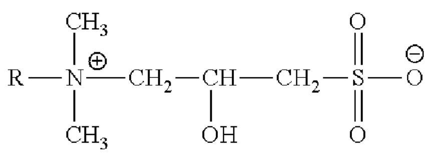

Hydroxysultaïnes d'alkyle
où R est un alkyle en $C_8$ à $C_{24}$, saturé ou insaturé, ou des mélanges de ceux-ci ;
iii) des amphoacétates d'alkyle

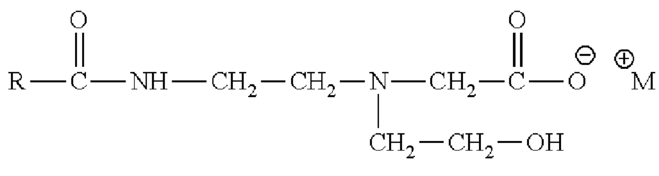

Amphoacétates d'alkyle
où R est un alkyle en $C_6$ à $C_{24}$ , saturé ou insaturé, ou des mélanges de ceux-ci et $M^+$ est un cation monovalent ; et
iv) des mélanges de ceux-ci.

**3.** Composition pour soins capillaires selon l'une quelconque des revendications précédentes, dans laquelle le ou les polyols sont choisis dans le groupe constitué de glycérine, éthylène glycol, propylène glycol, di-propylène glycol, éthylène glycol et des mélanges de ceux-ci, de préférence dans laquelle le ou les polyol(s) sont de la glycérine.

**4.** Composition pour soins capillaires selon l'une quelconque des revendications précédentes, comprenant de 10 à 28 % en poids de la composition pour soins capillaires d'un agent tensioactif anionique, de préférence de 10 à 20 % en poids de la composition pour soins capillaires d'un agent tensioactif anionique, encore plus préférablement de 10 à 15 % en poids de la composition pour soins capillaires d'un agent tensioactif anionique.

**5.** Composition pour soins capillaires selon l'une quelconque des revendications précédentes, comprenant de 2 % à 15 % en poids de la composition pour soins capillaires de l'agent tensioactif zwitterionique.

**6.** Composition pour soins capillaires selon l'une quelconque des revendications précédentes, dans laquelle l'agent tensioactif détersif est un parmi plus d'agents tensioactifs non ioniques.

**7.** Composition pour soins capillaires selon l'une quelconque des revendications précédentes, dans laquelle la composition pour soins capillaires contient en outre 0,05 à 5 % en poids d'un agent de conditionnement de silicone, de préférence dans laquelle l'agent de conditionnement de silicone contient un parmi un sel d'ammonium quaternaire dans sa structure moléculaire, encore plus préférablement dans laquelle l'agent de conditionnement de silicone est du diméthiconol.

**8.** Composition pour soins capillaires selon l'une quelconque des revendications précédentes, dans laquelle la composition pour soins capillaires comprend en outre de 0,05 à 2 % en poids de la composition pour soins capillaires d'un ou plusieurs polymères cationiques, de préférence dans laquelle les polymères cationiques sont choisis dans le groupe constitué de chlorure de guar hydroxypropyltrimonium, polyquaternium-6, polyquaternium-7, polyquaternium-10, polyquaternium-39, polyquaterinum-67, et des mélanges de ceux-ci.

**9.** Composition pour soins capillaires selon la revendication 8, dans laquelle le chlorure de guar hydroxypropyltrimonium a une masse moléculaire moyenne en poids allant de 50 000 à 1,0 million de g/mol, de préférence dans laquelle le chlorure de guar hydroxypropyltrimonium a une masse moléculaire moyenne en poids allant de 100 000 à 900 000 g/mol.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2042216 A1
- JP 2012001597 A
- US 2001056049 A1
- WO 02092050 A2
- WO 0142409 A1
- US 2006183662 A1
- DE 4315396 A1
- US 5747436 A
- WO 2016172404 A1
- US 6649155 B
- US 20080317698
- US 20080206355
- US 6335312 B, Coffindaffer
- US 2486921 A
- US 2486922 A
- US 2396278 A
- US 3929678 A
- US 2658072 A
- US 2438091 A
- US 2528378 A
- US 5104646 A
- US 5106609 A
- WO 2004078903 A
- WO 2004078901 A
- WO 2005078063 A


### Non-patent literature cited in the description


- **SEGUR, J.B. ; OBERSTAR, H.** *Ind. & Eng. Chem.,* 1951, vol. 43, 2117-2120
- **MCCUTCHEON'S.** Emulsifiers and Detergents. M. C. Publishing Co, 1989
- **SOLAREK, D. B.** Cationic Starches in Modified Starches: Properties and Uses. CRC Press, Inc, 1986, 113-125
- CTFA Cosmetic Ingredient Handbook. The Cosmetic, Toiletries, and Fragrance Association, Inc, 1988